# EUROPEAN PATENT APPLICATION

(11) **EP 2 100 597 A2**
(43) Date of publication of application: **16.09.2009**
(21) Application number: 08251224.5
(22) Date of filing: 31.03.2008
(51) Int. Cl.: A61K 9/127, A61K 47/48

(54) **Liposome complexes**

(30) Priority: 11.03.2008 US 46346
(71) Applicant: MEDTRONIC, INC., Minneapolis, MN 55432-5604 (US)
(72) Inventor: Benz, Michael E., Ramsey MN 55303 (US); Thakker, Deepak R., Blaine MN 55449 (US)
(74) Representative: Golding, Louise Ann

(57) **Abstract**

Liposome complexes are provided for site-specific delivery of pharmaceutical agents with improved targeting efficiency. The liposome complexes include a pharmaceutical agent comprising an SiRNA associated with the liposome; a sialic acid-containing molecule associated with the liposome; and optionally a targeting agent attached to the sialic acid-containing molecule. The invention also provides methods of making liposome complexes and *their in vivo* administration.

## Description

Liposomes, also known as vesicles, have been designed to encapsulate pharmacological agents useful for *in vivo* purposes such as the diagnosis and treatment of various diseases and conditions. These cargo-carrying liposomes have experimentally shown potential for being site-specific carrier systems for a variety of such agents. Agents so delivered to designated sites *in vivo* demonstrate significantly enhanced therapeutic indices. Concurrently, a decrease in unwanted side effects and wasted portions of a pharmaceutical dosage are achieved.

The advantages of encapsulation have been offset, however, by the deleterious effects of the body's reticulo-endothelial system (RES), mainly the liver and spleen. The reticulo-endothelial system acts to screen the body's circulation. The reticulo-endothelial system will gradually scavenge from the circulation all material it considers foreign and unwanted. Liposomes have certain physical characteristics which render them susceptible to removal by the reticulo-endothelial system. Once recognized, liposomes, whether given a site-specific molecule for so-called "targeted" delivery or not, are quickly phagocytosed by the reticulo-endothelial system along with their cargo.

From the foregoing, it will be appreciated that what is needed in the art is a liposome composition that delivers an encapsulated pharmaceutical agent into specific tissues (e.g., organs), particularly the brain, with reduced or no scavenging by the body's reticulo-endothelial system.

The present invention provides liposome complexes for site-specific delivery of pharmaceutical agents that include SiRNA and other optional pharmaceutical agents (e.g., therapeutic or diagnostic agents) to a targeted site with improved efficiency. Such liposome complexes include liposomes having associated therewith one or more types of sialic acid-containing molecules, preferably the sialic acid-containing molecules are attached to the external surface of the liposomes for surface modification. In certain preferred embodiments, the sialic acid-containing molecules form linkers for attachment of one or more targeting agents to the liposomes. In certain preferred embodiments, one or more pharmaceutical agents are located within the internal compartment of the liposome.

The liposome complexes of the present invention are particularly designed for delivering SiRNA and other optional pharmaceutical agents across the blood-brain barrier, although they can also be used to target other organs such as the heart, liver, kidney, pancreas, etc., or other tissues (e.g., skin, spine). The therapeutic management of almost all debilitating neurological and psychiatric disorders is largely hindered by the restricted access of pharmaceutical agents to the brain, imposed by the microvascular endothelial blood-brain barrier (BBB). A limited permeability of the BBB, only to pharmaceutical agents exhibiting high lipophilicity and molecular masses of less than 500 Daltons, mandates the use of craniotomy-based techniques for delivering other promising therapeutic candidates (e.g., injecting the pharmaceutical agent either directly into a specific site within the brain or into the ventricles carrying the cerebrospinal fluid). Besides the invasive nature of craniotomy, these techniques fail to accomplish widespread distribution of the pharmaceutical agent in the brain that can be attained using a trans-vascular approach. Current strategies, used for administering BBB-impermeable therapeutics via the trans-vascular route, involve co-infusion of a therapeutic with an osmotic agent or conjugation to vectors that enable an absorptive- or receptor-mediated transcytosis across the BBB. Such methods are generally undesirable because they allow for an adverse, non-specific influx of plasma constituents into the brain, generalized breakdown of the BBB, peripheral vascular permeability, generation of antibodies (antigenicity), generation of an immune response (immunogenicity), and/or inactivation of the pharmaceutical agent.

Preferred liposome complexes of the present invention enable a trans-vascular access and widespread distribution of SiRNA and optionally a broad range of pharmaceutical agents (preferably, therapeutic agents), including oligonucleotide-, amino acid- and chemical-based compounds, into the brain. In preferred embodiments, liposomal encapsulation protects the pharmaceutical agent from enzymatic degradation *in vivo.* In addition, in certain preferred embodiments, conjugation of anionic, non-immunogenic polysialic acids (PSAs) to the liposomal surface reduces and/or restricts the reticulo-endothelial uptake of the liposomes and extends their viable time in the bloodstream. In certain preferred embodiments, binding the liposomal PSAs to one or more targeting agents (e.g., monoclonal antibodies, antibody fragments, or peptide analogues that specifically recognize receptors expressed in the brain microvasculature) facilitates entry into the brain.

In one embodiment, the liposome complex includes: a liposome having an exterior surface and an internal compartment; a pharmaceutical agent comprising an SiRNA (and optionally other therapeutic and/or diagnostic agents) associated with the liposome (in certain embodiments, located within the internal compartment of the liposome); a sialic acid-containing (e.g., terminated) molecule associated with the liposome (in certain embodiments, the sialic acid-containing molecule is attached at the external surface of the liposome); and a targeting agent; wherein the sialic acid-containing molecule forms a linker between the targeting agent and the liposome (preferably, the external surface of the liposome).

In another embodiment, the liposome complex includes: a liposome having an exterior surface and an internal compartment; a pharmaceutical agent comprising an SiRNA (and optionally other therapeutic and/or diagnostic agents) associated with the liposome (in certain embodiments, located within the internal compartment of the liposome); a sialic acid-containing (e.g., terminated) molecule associated with the liposome (in certain embodiments, the sialic acid-containing molecule is attached at the external surface of the liposome); and optionally a targeting agent; wherein the sialic acid-containing molecule comprises polysialic acid having a degree of polymerization of at least 8 (particularly when the complex does not include a targeting agent).

In certain embodiments, the liposome is prepared from phospholipids selected from the group consisting of phosphatidylserine, phosphatidylinositol, phosphatidylethanolamine, phosphatidylcholines, phosphatidylglycerol, phosphatidic acid, phosphatidylmethanol, cardiolipin, ceramide, cholesterol, cerebroside, lysophosphatidylcholine, D-erythrosphingosine, sphingomyelin, dodecyl phosphocholine, N-biotinyl phosphatidylethanolamine, and combinations thereof.

In certain embodiments, at least one sialic acid-containing molecule includes a lipid head-group (e.g., a phospholipid) and a sialic acid group, which is then used in the preparation of a liposome. In certain embodiments, the sialic acid-containing molecule does not need to be conjugated with a lipid; rather, it can be used in an unconjugated form and added to the liposome preparation. In certain embodiments, the sialic acid-containing molecule is selected from the group consisting of capsular polysialic acid, sialic acid-containing gangliosides, colominic acid (i.e., a homopolymer of N-acetylneuraminic acid (NANA) with α(2→8) ketosidic linkages), and combinations thereof. Preferably, the sialic acid-containing molecule is a capsular polysialic acid. In one embodiment, colominic acid can be pre-conjugated to 1,2-distearoyl-sn-glycero-3-phosphoethanolamine (DSPE) and used in the preparation of a liposome. Alternatively, colominic acid can be used in the preparation of a liposome without pre-conjugating it to a lipid.

In certain embodiments, the targeting agent is selected from the group consisting of peptides, peptide analogs, antibodies, antibody fragments, small organic or inorganic molecules, and combinations thereof. Preferably, the targeting agent is a compound that specifically recognizes receptors expressed in the brain microvasculature.

In certain embodiments, the liposome complex includes two or more different pharmaceutical agents.

The present invention also provides a pharmaceutical composition that includes a liposome complex of the present invention and an optional pharmaceutically acceptable carrier. In certain embodiments, a pharmaceutical composition can also include a pharmaceutical agent that is not a part the liposome complex, which may be the same or different than the pharmaceutical agent associated with the liposome.

In certain embodiments, a pharmaceutical composition can crosslink, gel, or change in viscosity on or after administration to a subject. In certain embodiments, a pharmaceutical composition can provide for delayed or extended release of the liposome complex after administration to a subject.

In certain embodiments, a pharmaceutical composition can include two or more different liposome complexes, each containing different pharmaceutical agents and/or targeting moieties.

The present invention also provides methods of treating and/or preventing an affliction by administering a liposome complex of the present invention or pharmaceutical composition containing such complex to a subject. The affliction can be a psychiatric disorder, a neurological disorder, a disease affecting and/or originating in the brain, a disorder of the cardiac system, a disorder of the hepatic system, a disorder of the vascular system, a disorder of the orthopedic system, or combinations thereof.

The present invention also provides methods of delivering a pharmaceutical agent to a subject by administering a liposome complex of the present invention or pharmaceutical composition containing such complex to the subject.

The present invention also provides methods of making a liposome complex. One method includes: preparing a liposome having an exterior surface and an internal compartment, a pharmaceutical agent comprising an SiRNA associated therewith, and a sialic acid-containing molecule associated therewith; and attaching a targeting agent to the sialic acid-containing molecule; wherein sialic acid-containing molecule forms a linker between the targeting agent and the liposome (preferably, the external surface of the liposome).

Another method includes: preparing a liposome having an exterior surface and an internal compartment, a pharmaceutical agent comprising an SiRNA associated therewith, and a sialic acid-containing molecule associated therewith; and optionally attaching a targeting agent to the sialic acid-containing molecule; wherein the sialic acid-containing molecule comprises polysialic acid having a degree of polymerization of at least 8.

In one method, preparing a liposome having an exterior surface and an internal compartment and a pharmaceutical agent associated therewith involves encapsulating the pharmaceutical agent within the internal compartment of the liposome. Preferably, the step of preparing a liposome having an exterior surface and an internal compartment and a sialic acid-containing molecule associated therewith involves attaching a sialic acid-containing molecule to the external surface of the liposome during or after encapsulation of the pharmaceutical agent. Preferably, the method further includes a step of attaching a targeting agent to the sialic acid-containing molecule before, during, or after attaching the sialic acid-containing molecule to the external surface of the liposome.

In certain embodiments of the method, preparing a liposome includes forming the liposome from a sialic acid-containing molecule that includes a lipid group and a sialic acid group. In other embodiments, preparing a liposome includes forming the liposome from a sialic acid-containing molecule that does not include a lipid group.

### Definitions

As used herein, "sialic acid-containing molecule associated with the liposome" means that such molecules are included within the structure of the liposome, through covalent bonds or through non-covalent interactions. For example, a polysialic acid unit could be incorporated into a liposome through non-bonded interactions by the presence of a lipid or lipid-like head group, as is found in the naturally occurring capsular polysialic acids.

As used herein, "pharmaceutical agent associated with the liposome" means that such agent can be included within the structure of the liposome in a variety of manners. For example, association can be accomplished through encapsulation within the internal compartment of the liposome, or through attachment to the outer surface of the liposome through bonding or nonbonding interactions, or through intercalation between the double layer of lipid head groups, or through other methods known in the art of drug delivery using liposomes.

As used herein, "liposome" refers to a vesicle (generally spherical in shape) in an aqueous medium, formed by a lipid bilayer enclosing an aqueous compartment.

As used herein, "sialic acid" refers to the N-acyl derivative of neuraminic acid.

As used herein, "targeting agent" refers to a compound that binds to a specific site in the body.

As used herein, "pharmaceutical agent" refers to a therapeutic agent and/or a diagnostic agent.

As used herein, the term "comprises" and variations thereof do not have a limiting meaning where these terms appear in the description and claims. As used herein, "a," "an," "the," "at least one," and "one or more" are used interchangeably. Thus, for example, a liposome complex that comprises "a" pharmaceutical agent can be interpreted to mean that the liposome complex includes "one or more" pharmaceutical agents.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

Also herein, the recitations of numerical ranges by endpoints include all numbers subsumed within that range (e.g., 1 to 5 includes 1, 1.5, 2, 2.75, 3, 3.80, 4, 5, etc.).

The term "and/or" means one or all of the listed elements (e.g., preventing and/or treating an affliction means preventing, treating, or both treating and preventing further afflictions).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

The invention provides a liposome complex for delivering a pharmaceutical agent including SiRNA and optionally one or more other pharmaceutical agents (e.g., a diagnostic and/or therapeutic agent, and preferably a therapeutic agent), to a targeted area in a subject. The liposome complex includes a liposome having an exterior surface and an internal compartment, at least one pharmaceutical agent comprising SiRNA associated with the liposome (in certain embodiments, one or more pharmaceutical agents, at least one of which is an SiRNA are located within the internal compartment of the liposome), and at least one sialic acid-containing molecule that is associated with the liposome.

In certain embodiments, the liposome complex also includes at least one targeting agent, wherein the at least one targeting agent binds to a specific site in the body of the subject and is attached to (e.g., covalently bonded to) the at least one sialic acid-containing molecule (i.e., the sialic acid-containing molecule forms a linker between the targeting agent and the liposome, preferably the external surface of the liposome).

Liposome complexes of the present invention can be used to target various organs (e.g., brain, heart, liver, kidney, pancreas) or other tissues of the body (e.g., skin, spine). For example, a liposome complex can be administered to a subject for preferential uptake by an organ of interest.

The liposome complexes of the present invention are particularly designed for delivering pharmaceutical agents across the blood-brain barrier. As discussed above, the blood-brain barrier (BBB) poses a central problem in the unresponsiveness of almost all debilitating neurological disorders to conventional drug therapy.

The liposomes can form nanocontainers, such as nanoparticles, and are commonly used for encapsulation of pharmaceutical agents. They are typically spherical in shape, and preferably have an average particle size (i.e., the average of the longest dimension, which is the diameter for spherical particles) of no greater than 1000 nanometers (nm). Liposomes having an average particle size of 50 nm are desired, although other sizes may also be useful for crossing the blood brain barrier.

Suitable types of liposomes may be prepared from, for example, phospholipids selected from the group consisting of phosphatidylserine, phosphatidylinositol, phosphatidylethanolamine, phosphatidylcholines, phosphatidylglycerol, phosphatidic acid, phosphatidylmethanol, cardiolipin, ceramide, cholesterol, cerebroside, lysophosphatidylcholine, D-erythrosphingosine, sphingomyelin, dodecyl phosphocholine, N-biotinyl phosphatidylethanolamine, synthetic analogs of these molecules, derivatives of these molecules, and combinations thereof.

Liposomes may be prepared according to any of the well known conventional processes. For example, liposomes may be made by depositing a thin film of lipid on the inner wall of a flask, adding an aqueous phase, and shaking vigorously (e.g., by hand). Another method may include, for example, sonication of a lipid film in an aqueous solution, followed by extrusion through a series of filters (e.g., of decreasing pore size). Yet another method of making liposomes is to dialyze an aqueous solution of lipids in the presence of a detergent such as sodium cholate. As the detergent is depleted, the lipids form liposomes. Still another method is based on high pressure homogenization of a lipid solution using commercially available equipment. Additional methods may include, for example, re-hydration of freeze-dried vesicles and reverse-phase evaporation. Descriptions and protocols for these methods are well known to those of skill in the art. See, for example, Liposomes: A Practical Approach (2nd edition, 2003), edited by Vladimir Torchilin and Volkmar Weissig, Oxford University Press, Oxford, UK. Materials for making liposomes are commercially available, for example, from Avestin Inc., Ottawa, Canada, Microfluidics, a division of MFIC Corp., Newton, MA, and Harvard Apparatus, a Harvard Bioscience, Inc. Company, Holliston, MA.

One or more pharmaceutical agents, at least one of which is an SiRNA, may be associated with a liposome, such as encapsulated within a liposome, using a wide variety of mechanisms, including encapsulation within the internal compartment of the liposome, or attachment to the outer surface of the liposome through bonding or nonbonding interactions, intercalation between the double layer of lipid head groups, and the like. For example, methods of associating one or more pharmaceutical agents with liposomes include, but are not limited to: encapsulating an agent within the aqueous core of the liposome, which can occur by preparing the liposome in the presence of the agent; causing a non-bonded interaction (e.g., van der Waals) between an agent and the hydrophilic tail of a lipid used to form the liposome, either within the core or at the outer surface of the liposome; causing an interaction between an agent and the lipid head group of a lipid used to form the liposome; intercalating an agent between the double layer of lipid head groups in a liposome; bonding (e.g., covalent, ionic, or hydrogen bonding) an agent to a molecule that makes up the structure of the liposome, possibly through either the hydrophobic tail or the lipid head group; and/or causing complex formation between an agent and a cationic salt that may be a part of the structure of the liposome.

The pharmaceutical agents employed, at least one of which is an SiRNA, do not impose any significant limitation upon the scope of the invention. A wide variety of agents can be used. Such agents that are particularly susceptible to liposomal entrapment or association are useful. Such agents can be for therapeutic and/or diagnostic purposes. Such agents include oligonucleotide-based compounds (e.g., DNA, RNA, and SiRNA), amino acid-based compounds (e.g., proteins and peptides), polysaccharides, small molecule organic and inorganic compounds, organometallic species, and the like. Various combinations of pharmaceutical agents may be incorporated into liposomes according to the present invention.

Therapeutic agents include, for example, antibiotics, antidepressants, antitumorigenics, antivirals, cytokines, hormones, imaging agents, neurotransmitters, nucleic acids, stimulants, regulating agents that turn genes and/or protein production on or off, and the like. Suitable therapeutic agents may include, for example, poly-functional alkylating agents, such as mechlorethamine, chlorambucil, melphalan, thiotepa, busulfan, cyclophosphamide, ifosfamide; antimetabolites, such as methotrexate, 6-mercaptopurme, 6-thioguanme, 5-fluorouracil, 5-fluorodeoxyuridine, cytarabine, fludarabine, 2-chlorodeoxyadenosine, 2-deoxycoformycin, genicitabine: antibiotics, such as doxorubicin, bleomycin, dactinomycin, daunorubicin, plicamycin, mitomycin C, mitoxantrone; steroid and hormonally active compounds such as the androgen, fluoxymesterone; the antiandrogen, flutamide; the estrogens, ethinyl estradiol and diethylstilbestrol; the antiestrogen, tamoxifen; the progestational agent, megestrol acetate; the luteinizing hormone-releasing hormone agonist, leuprolide; the aromatase inhibitor, aminoglutethimide; the adrenal cortical compound, dexamethasone; miscellaneous drugs, such as asparaginase, altretaimine, carmustine, lomustine, steptozotocin, mitotane, dacarbazine, hydroxyurea, etoposide, cisplatin, carboplatin, procarbazine, vinblastine, vincristine, levamisole, cis-retinoic acid, paclitaxel, docetaxel; and biologic agents, such as α-interferon, β-interferon, interferon-y tumor necrosis factor, erythropoietin, granulocyte colony-stimulating factor, granulocyte macrophage colony-stimulating factor, macrophage colony-stimulating factor, interleukin-1, interleukin-2; and combinations thereof.

Diagnostic agents include, for example, metals (e.g., gadolinium) that can be detected by MRI, agents for enhancing radiopacity (e.g., barium), radioisotopes, and the like. Techniques of using liposomes with diagnostic agents are known in the art and described, for example, in Arti, J.Clin. Oncol., 24, 3299-3308 (2006).

SiRNA refers to "small interfering RNA," by which is meant a polynucleotide that has complementarity in a substrate binding region to (i.e., able to base-pair with) a specified gene target, and which acts to specifically guide enzymes in the host cell to cleave the target RNA (generally messenger RNA). That is, the small interfering RNA by virtue of the specificity of its sequence and its homology to the RNA target, is able to cause cleavage of the RNA strand and thereby inactivate a target RNA molecule because it is no longer able to be transcribed.

SiRNA is used to trigger a cellular reaction known as RNA interference. In RNA interference, double-stranded RNA is digested by an intracellular enzyme known as Dicer, producing SiRNA duplexes. The SiRNA duplexes bind to another intracellular enzyme complex, which is thereby activated to target whatever mRNA molecules are homologous (or complementary) to the SiRNA sequence. The activated enzyme complex cleaves the targeted mRNA, destroying it and preventing it from being used to direct the synthesis of its corresponding protein product. Recent evidence suggests that RNA interference is an ancient, innate mechanism for not only defense against viral infection (many viruses introduce foreign RNA into cells) but also gene regulation at very fundamental levels. RNA interference has been found to occur in plants, insects, lower animals, and mammals, and has been found to be dramatically more effective than other gene silencing technologies, such as antisense or ribozyme technologies.

The complementary regions allow sufficient hybridization of the small interfering RNA to the target RNA and thus permit cleavage. One hundred percent complementarity is often necessary for biological activity and therefore is preferred, but complementarity as low as 90% may also be useful in this invention (such as at least 91%, at least 92%, at least 93%, at least 94%, at least 95%, at least 96%, at least 97%, at least 98%, or at least 99%). For example, 19 bases out of 21 bases may be base-paired. In some instances, where selection between various allelic variants is desired, 100% complementary to the target gene is required in order to effectively discern the target sequence from the other allelic sequence. When selecting between allelic targets, choice of length is also an important factor because it is the other factor involved in the percent complementary and the ability to differentiate between allelic differences.

The specific small interfering RNA described in the present application are not meant to be limiting and those skilled in the art will recognize that all that is important in a small interfering RNA is that it have a specific substrate binding site which is complementary to one or more of the target nucleic acid regions.

Use of SiRNA methodology involves introducing into cells (or causing cells to produce) short, double-stranded molecules of RNA similar to those that would be produced by the Dicer enzyme from an invading double-stranded RNA virus. The artificially-triggered RNA interference process then continues from that point.

To deliver a small interfering RNA to a subject, a preferred method involves introducing DNA encoding for the SiRNA, rather than the SiRNA molecules themselves, into target cells. The DNA sequence encoding for the particular therapeutic SiRNA can be specified upon knowing (a) the sequence for a small and accessible portion of the target mRNA (available in public human genome databases, particularly the NCBI database), and (b) well-known scientific rules for how to specify DNA that will result in production of a corresponding RNA sequence when the DNA is transcribed by cells. The DNA sequence, once specified, can be constructed in the laboratory from synthetic molecules ordered from a laboratory supplier, and inserted using standard molecular biology methods into one of several alternative vectors for delivery of DNA to cells. Once delivered into the target cells, those cells will themselves produce the RNA that becomes the therapeutic SiRNA, by transcribing the inserted DNA into RNA. The result will be that the cells themselves produce the SiRNA that will silence the targeted gene. The result will be a reduction of the amount of the targeted protein produced by the cell.

Thus, also within the scope of the present invention is the use of specifically tailored vectors designed to deliver small interfering RNA directly to targeted cells. The success of the designed small interfering RNA is predicated on their successful delivery to the targeted cells.

Small interfering RNA molecules have been shown to be capable of targeting specific mRNA molecules in human cells. Small interfering RNA vectors can be constructed to transfect human cells and produce small interfering RNA that cause the cleavage of the target RNA and thereby interrupt production of the encoded protein.

In the present invention, the small interfering RNA are targeted to complementary sequences in the mRNA sequence coding for the production of the target protein, either within the actual protein coding sequence, or in the 5' untranslated region or the 3' untranslated region. After hybridization, the host enzymes guided by the SiRNA are capable of cleavage of the mRNA sequence. Perfect or a very high degree of complementarity is typically needed for the small interfering RNA to be effective. A percent complementarity indicates the percentage of contiguous residues in a polynucleotide that can form hydrogen bonds (e.g., Watson-Crick base pairing) with a second polynucleotide (e.g., 5, 6, 7, 8, 9, 10 out of 10 being 50%, 60%, 70%, 80%, 90%, and 100% complementary). "Perfectly complementary" means that all the contiguous residues of a polynucleotide will hydrogen bond with the same number of contiguous residues in a second polynucleotide. However, it should be noted that single mismatches, or base-substitutions, within the SiRNA sequence can substantially reduce the gene silencing activity of a small interfering RNA.

The small interfering RNA sequence needs to be of sufficient length to bring the small interfering RNA and target RNA together through complementary base-pairing interactions. By "sufficient length" is meant a polynucleotide that is of a length great enough to provide the intended function under the expected condition. The small interfering RNA of the invention may be of varying lengths. The length of the small interfering RNA is preferably greater than or equal to ten nucleotides and of sufficient length to stably interact with the target RNA. By "stably interact" is meant interaction of the small interfering RNA with a target polynucleotide (e.g., by forming hydrogen bonds with complementary nucleotides in the target under physiological conditions). In preferred embodiments of the present invention, a small interfering RNA is 15 to 30 nucleotides in length. In particular embodiments, the polynucleotide is 15, 16, 17, 18, 19, 20, 21, 22, 23, 24, 25, 26, 27, 28, 29, or 30 nucleotides in length. In preferred embodiments the length of the siRNA sequence can be between 19-30 base pairs, and more preferably between 21 and 25 base pairs, and more preferably between 21 and 23 base pairs.

In certain embodiments, the number of pharmaceutical agents associated with (e.g., encapsulated within) the liposomes may vary from one to many, depending on the desired result (e.g., the affliction being treated). In certain embodiments, liposome complexes may include at least one pharmaceutical agent at a suitable level to produce the desired result.

One or more sialic acid-containing molecules are associated with liposomes according to the present invention. This may be through a covalent bond, a non-covalent bond, or other mechanism of interaction or association. For example, a sialic acid-containing molecule can be conjugated to a polar lipid head group or other lipid component of a liposome. Alternatively, an unconjugated sialic acid-containing molecule can be combined with the components used to make a liposome.

In certain embodiments, the sialic acid-containing molecule(s) are attached to the liposome (preferably, the external surface of the liposomes) after the liposomes are prepared. Alternatively, sialic acid-containing molecules, such as polysialic acids, can be used to make the liposomes and thereby be incorporated into the structure of the liposome during preparation of the liposome. Various combinations of such mechanisms of associating sialic acid-containing molecules can be used in any one liposome complex.

As a result of association of sialic acid-containing molecule(s), particularly through surface modification of the liposomes, the liposome complexes of the present invention experience less scavenging by the body's reticulo-endothelial system than occurs with liposomes that do not have sialic acid-containing molecules associated therewith. While not being limited to theory, it is believed that while the liposomal encapsulation reduces and even prevents enzymatic degradation of the pharmaceutical *agent in vivo,* the hydrophilic nature of the sialic acid-containing molecule forms a protective "watery" coating around the liposome, thereby providing protection from reticulo-endothelial uptake. Advantageously, this combination of liposome and sialic acid surface modification increases the blood residence time of the pharmaceutical agent. Also, by virtue of their endogenous expression in mammals, sialic acid-containing molecules present a negligible risk of generating antigenic or immunogenic responses and are generally biodegradable, in contrast to currently employed polymers such as poly(ethylene glycol).

In certain embodiments, the sialic acid-containing molecules may be used to link one or more targeting agents to the liposome (preferably, the external surface of the liposome). Alternatively, one or more targeting agents can be directly attached to the liposomes or attached to linkers other than sialic acid-containing molecules. Various combinations of attaching targeting agents to the liposomes can be used in any one liposome complex.

The sialic acid-containing molecule, may include, for example, a wide variety of materials that include sialic acid, polysialic acid, sialic acid analogues and polysialic acid analogues (such analogues are materials containing synthetically modified sialic acid), phospholipids and polysaccharides containing sialic acid units (including those containing polysialic acid at a chain terminus), and the like. Various combinations of different sialic acid-containing molecules can be used if desired.

In certain embodiments, the sialic acid-containing molecule may be oligomeric or polymeric and include at least 2 sialic acid units, more preferably at least 4 sialic acid units, and even more preferably at least 8 sialic acid units. Such materials are also referred to as "polysialic acids," or polymers of sialic acid whose degree of polymerization (DP) is preferably at least 2, more preferably at least 4, and even more preferably at least 8. There is no necessary limitation to the upper limit of the number of sialic acid units. Generally, such polymers could be as large as naturally occurring polysialic acids. In some embodiments, the number of sialic acid units is no greater than 1000, and in some embodiments no greater than 200.

In certain embodiments, the sialic acid-containing molecule includes, for example, a lipid head-group and a sialic acid group. The lipid head-group may be a terminal phospholipid group for insertion into the lipid layer. Suitable terminal phospholipids groups may include, for example, those listed above, and combinations thereof.

Suitable examples of sialic acid-containing molecules may include, for example, capsular polysialic acid, sialic acid-containing gangliosides, colominic acid (i.e., a polysialic acid in which all sialic acid residues are linked in 2→8 fashion), and combinations thereof. Preferably, the sialic acid-containing molecules are anionic polymers of N-acetylneuraminic acid.

Capsular polysialic acids are generally referred to by reference to the microorganisms that produce them. Suitable examples include Serogroup B capsular polysialic acid B from N. meningitidis or E. coli K1, Serogroup C capsular polysialic acid from N. meningitidis C, and polysialic acid from E. coli K92, which are abbreviated as PSB, PSC, and PSK92, respectively. See, for example, Gregoriadis et al., FEBS Letter, 315(3), January 1993, pp 271-76. Synthetic analogs of capsular polysialic acid are also suitable. One such analog is described as a twin-tailed, lipid-linked polysialic acid in Matthews et al., Biopolymers, 33, pp 453-7 (1993).

Sialic acid-containing gangliosides are commercially available from sources such as Avanti Polar Lipids, Alabaster, AL. One such product has the structure 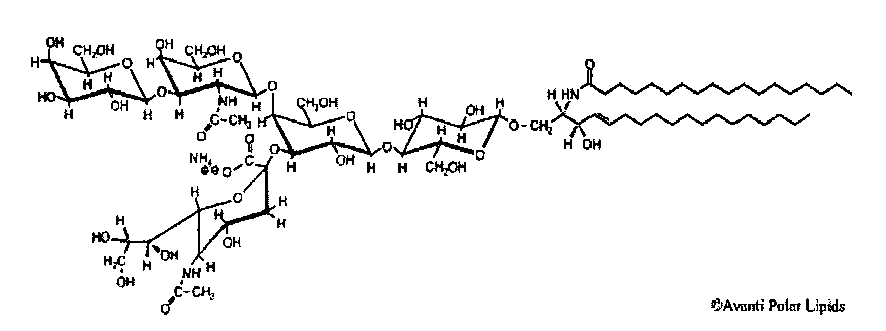 and is referred to by Product Number 860065; G_{M1} (Ovine); C_{M1} Ganglioside (Brain, Ovine-Ammonium Salt); or GalBetal-3GalNAcBetal-4(NeuAcAlpha2-3)GalBetal-4GlcBetal-1'-Cer (Brain, Ovine-Ammonium Salt). Mixtures of sialic acid-containing gangliosides are also commercially available.

Attachment of the sialic acid-containing molecule(s) to the liposome (preferably, the external surface of the liposome) can occur during or after encapsulation of the pharmaceutical agent(s). Suitable methods for attaching the sialic acid-containing molecule to the liposome may include, for example, the method described in the articles described above.

In certain embodiments, the liposome complexes of the present invention may include at least one sialic acid-containing molecule at a suitable level to produce the desired result. More than one type of sialic acid-containing molecule can be used if desired.

In certain preferred embodiments, it is desirable to also include targeting agents in the liposome complexes. For example, to provide transport of the liposomes that contain pharmaceutical agent(s) across the blood-brain barrier (BBB), a number of blood-brain targeting agents may be attached to the sialic acid-containing molecule(s).

Preferably, the targeting agent is selected from the group consisting of peptides, peptide analogs, antibodies (whether monoclonal or polyclonal) or fragments thereof, small organic or inorganic or organometallic molecules (i.e., compounds or portions thereof), and combinations thereof. More preferably, the targeting agent is a compound or a portion thereof that specifically recognizes receptors expressed in the brain microvasculature (e.g., receptors for bradykinin, insulin, insulin-like growth factors, leptin, low-density lipoproteins, and transferrin). Targeting agents may be endogenous peptide ligands of the receptors, analogs of the endogenous ligands, or peptidomimetic monoclonal antibodies (MAbs such as 8D3, R17, and OX26 antibodies) that bind the same receptor of the endogenous ligand. See, for example, Shusta, NeuroRx, 2, 151-161 (2005); Pardridge et al., Pharmaceutical Research, 12, 807-816 (1995); and Lee et al., Journal of Pharmacology and Experimental Therapeutics, 292, 1048-1052 (2000).

Suitable targeting agents may include, for example, insulin, transferrin, insulin-like growth factors, and leptin. Such compounds or portions thereof facilitate the receptor-mediated transcytosis of the liposome complexes of the present invention across the BBB, and subsequent entry into the brain.

If desired, a molecule that includes sialic acid could be conjugated with two different targeting agents, one peptide targeting an endogenous blood-brain barrier receptor (such as those discussed above) and the other targeting an endogenous brain cell membrane peptide. The latter could be specific for particular cells within the brain, such as neurons, glial cells, pericytes, smooth muscle cells, or microglia. The latter could also be added to enhance the cellular uptake, e.g., cell-penetrating peptides that include those described in Dietz et al., Mol. & Cell. Neurosci., 27, 85-131 (2004). Accordingly, therapeutic delivery can further be exacted to specific cell types by targeting the receptors expressed on these cells within the brain (or other organ, for example).

Attachment of the targeting agent(s) to the liposome (preferably, external surface of the liposome) can occur before, during, or after the sialic acid-containing molecule(s) are associated with to the liposomes. Suitable methods for attaching the targeting agent(s) to the sialic acid-containing molecule(s) may include, for example, generation of an aldehyde on the terminal sialic acid unit (e.g., by reaction with sodium periodate), which may react with an amine of the targeting agent to yield an imine. This imine may be subsequently reduced by a reducing agent such as sodium borohydride to yield a hydrolytically stable secondary or tertiary amine.

In certain embodiments, the liposome complexes of the present invention may include at least one targeting agent at a suitable level to produce the desired result. More than one type of targeting agent can be used if desired.

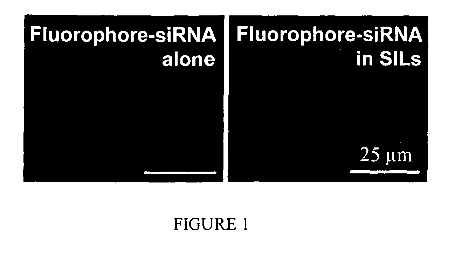

Figure 1 shows a particular example of one embodiment of the present invention in which a liposome complex encapsulates an SiRNA.

The invention also provides methods of making liposome complexes for delivering a pharmaceutical agent, at least one of which is an SiRNA. As discussed herein, such methods typically involve preparing a liposome having an exterior surface and an internal compartment and associating a pharmaceutical agent with the liposome (preferably, incorporating a pharmaceutical agent within the internal compartment of the liposome). A sialic acid-containing molecule can be associated with (e.g., attached at the external surface of) the liposome during or after association of the pharmaceutical agent. If used, a targeting agent can be attached to the sialic acid-containing molecule before, during, or after association of the sialic acid-containing molecule with the liposome.

The invention also provides methods *of in vivo* administration of a liposome complex of the present invention for delivering a pharmaceutical agent, at least one of which is an SiRNA, to a subject. In one embodiment, such administration could be used for treating and/or preventing one or more afflictions such as a psychiatric or neurological disorder or any disease affecting and/or originating in the brain such as Parkinson's Disease and Alzheimer's Disease, or disorders of one or more of the cardiac, hepatic, vascular, or orthopedic systems.

Various combinations of liposome complexes could be used, each containing a different pharmaceutical agent, for desired effect. Alternatively, various combinations of pharmaceutical agents (not associated with a liposome) and liposome complexes of the present invention could be administered to a subject. For example, a pharmaceutical agent could be added to the delivery solution that contains a liposome complex of the present invention.

Preferably, the liposome complex is administered intra-vascularly and crosses the blood brain barrier, which is particularly desirable for preventing and/or treating neurological disorders.

The liposome complexes of this invention provide useful mechanisms for pharmaceutical applications for administering a therapeutic or diagnostic agent to a subject. Accordingly, the liposome complexes of this invention are useful as pharmaceutical compositions, optionally in combination with pharmaceutically acceptable carriers. Such pharmaceutical compositions can include two or more types of liposome complexes, each containing different pharmaceutical agents and/or targeting moieties. Such pharmaceutical compositions can also include one or more pharmaceutical agents that are not a part of the liposome complex.

Liposome complexes of the present invention can be included in pharmaceutical compositions that crosslink, gel, or change in viscosity on or after administration to a subject. This can occur, for example, through covalent bond formation, hydrogen bond formation, pH change, temperature change, complex formation, and the like.

Liposome complexes of the present invention can be included in pharmaceutical compositions that provides for delayed or extended release of the liposome complex, and, hence, the pharmaceutical agent(s) associated therewith after administration to a subject. This can occur, for example, using pH sensitivity as a trigger for release.

Administration of the liposome complexes described herein can be via any of the accepted modes of administration for the biologically active substances that are desired to be administered. These methods include oral, topical, parenteral, ocular, transdermal, nasal and other systemic or aerosol forms, although IV administration is preferred.

Depending on the intended mode of administration, the compositions used may be in the form of solid, semi-solid or liquid dosage forms, such as, for example, tablets, suppositories, pills, capsules, powders, liquids, suspensions, or the like, preferably in unit dosage forms suitable for single administration of precise dosages. The pharmaceutical compositions will include the liposome complexes as described herein and optionally a pharmaceutical acceptable excipient, other medicinal agents, pharmaceutical agents, carriers, adjuvants, etc.

Topical formulations composed of the liposome complexes described herein, penetration enhancers, and other biologically active drugs or medicaments can be applied in many ways. The solution can be applied dropwise, from a suitable delivery device, to the appropriate area of skin or diseased skin or mucous and be integrated over a total time period of the sustained-release device in order to compute the appropriate dose required. Although effective dosage ranges for specific biologically active substances of interest are dependent upon a variety of factors, and are generally known to one of ordinary skill in the art, some dosage guidelines can be generally defined.

In general, topical formulations are prepared in gels, creams, or solutions having an active ingredient in the range of from 0.001 % to 10% (weight by volumne (w/v)), preferably 0.01 to 5%, and most preferably about 1% to about 5%. Of course, these ranges are subject to variation depending upon the potency of the therapeutic agent, and could in appropriate circumstance fall within a range as broad as from 0.001 % to 20%. In all of these exemplary formulations, as well as other topical formulations, the total dose given will depend upon the size of the affected area of the skin and the number of doses per day. The formulations may be applied as often as necessary, but preferably not more than about three times per day.

For oral administration, a pharmaceutically acceptable, non-toxic composition is formed by the incorporation of any of the normally employed excipients, such as, for example, mannitol, lactose, starch, magnesium stearate, sodium saccharine, talcum, cellulose, sodium crosscarmellose, glucose, gelatin, sucrose, magnesium carbonate, and the like. Such compositions include solutions, suspensions, tablets, dispersible tablets, pills, capsules, powders, sustained-release formulations, and the like.

Preferably the compositions will take the form of a pill or tablet. Thus the composition will contain along with the active ingredient: a diluent such as lactose, sucrose dicalcium phosphate, and the like; a lubricant such as magnesium stearate, and the like; and a binder such as starch, gum acacia, gelatin, polyvinylpyrrolidone, cellulose and derivatives thereof, and the like.

Liquid pharmaceutically administrable compositions can, for example, be prepared by dissolving, dispersing, etc., the liposomes as described above and optional pharmaceutical adjuvants in a carrier, such as, for example, water, saline, aqueous dextrose, glycerol, glycols, ethanol, and the like, to thereby form a suspension. If desired, the pharmaceutical composition to be administered may also contain minor amounts of nontoxic auxiliary substances such as wetting agents, emulsifying agents, or solubilizing agents, pH buffering agents, and the like, for example, acetate, sodium citrate, cyclodextrin derivatives, sorbitan monolaurate, triethanolamine sodium acetate, triethanolamine oleate, etc.

Actual methods of preparing such dosage forms are known, or will be apparent, to those skilled in this art. The composition or formulation to be administered will, in any event, contain a quantity of the active pharmaceutical agent in an amount sufficient to effectively treat the disorder or disease of the subject being treated.

For a solid dosage form, the suspension, in, for example, propylene carbonate, vegetable oils or triglycerides, is preferably encapsulated in a gelatin capsule. Such suspensions and the preparation and encapsulation thereof, can be prepared by methods that are well known to those of skill in the art. For a liquid dosage form, the suspension may be diluted with a sufficient quantity of a pharmaceutically acceptable liquid carrier, e.g., water, to be easily measured for administration.

Alternatively, liquid or semi-solid oral formulations may be prepared by dissolving or dispersing the liposome complexes in vegetable oils, glycols, triglycerides, propylene glycol esters (e.g., propylene carbonate), and the like, and encapsulating these solutions or suspensions in hard or soft gelatin capsule shells.

Nasal solutions of the liposome complexes alone or in combination with pharmaceutically acceptable excipients can also be administered.

Formulations of the liposome complexes may also be administered to the respiratory tract as an aerosol for a nebulizer. In such a case, the particles of the formulation have diameters of less than 50 microns, preferably less than 10 microns.

Intravenous administration of the liposome complexes may also be used.

The efficacy of delivery of the liposome complexes with one or more pharmaceutical agents is generally determined by the targeted tissue. For example, in targeting the blood brain barrier, complexes of the present invention that include a targeting antibody that recognizes the transferrin receptor (known to be expressed at high levels in the blood brain barrier) can be injected into a catheter that is pre-implanted into the carotid artery or by a tail vein injection. In targeting striatal neurons, complexes of the present invention that include a targeting antibody that recognizes the dopamine D1 receptor (known to be expressed at high levels in striatal neurons) can be injected intra-cranially into the striatum.

Objects and advantages of this invention are further illustrated by the following example, but the particular materials and amounts thereof recited, as well as other conditions and details, should not be construed to unduly limit this invention. In the following example, reference is made to accompanying FIG. 1 which is a fluorescent image that depicts the siRNA/liposome complexes as punctate red fluorescent particles (signal emitting from the DY-547 red fluorescent label on siRNA shown as lighter spots in the image) when imaged under a fluorescent microscope.

### Example 1: Preparation of Liposomes with SiRNA

Liposomes composed of POPC (1-palmitoyl-2-oleoyl-sn-glycerol-3-phosphocholine), DDAB (didodecyldimethylammonium bromide), and ganglioside were prepared at a ratio of 82:15:3. Sterile RNase-free equipment was used for the entire procedure.

Lipid films were first created and then rehydrated. Each lipid was separately dissolved in chloroform:methanol at a ratio of 2:1 in a 10 mL round-bottomed flask with a 14/20 outer joint as follows: Approximately 10 mg of octyl-β-D-glucopyranoside was added to each flask. In one flask, 3 µmol (0.1 mL a 10 mg/mL stock solution) of DDAB in chloroform (Avanti Polar Lipids), and 100 µL each of chloroform and methanol were added to maintain the chloroform:methanol ratio of 2:1. In a second flask, 16.4 µmol (or 0.621 mL of a 10 mg/mL stock solution) of POPC in chloroform (Avanti Polar Lipids, Alabaster, AL) and 0.3105 mL methanol were added in order to maintain the chloroform:methanol ratio of 2:1.

Approximately 0.6 µmol (0.55 mg) of ganglioside (EMD Biosciences, Gibbstown, NJ) was added to the POPC-containing flask. Each lipid mixture was carefully vortexed using moderate speed for 30 seconds. The majority of the solvent in each flask was removed by blowing nitrogen gas over the flask surface. The remainder of the solvent was removed using a rotary evaporator to make white lyophilized lipid film. Each lipid film was then separately re-suspended and dispersed in 250 µL of buffer (20 mM HEPES and 140 mM NaCl, pH 7.5) made with DEPC-H₂O Each mixture was vortexed gently to cause each lipid to go completely into suspension (this can take 2-5 minutes). Following this procedure, each re-suspended lipid/buffer mixture was sonicated for 10 minutes, and vortexed with moderated speed for 30 seconds.

To the flask containing the cationic (i.e., containing DDAB) lipid solution, si*GLO* Red RISC-free siRNA (Dharmacon; Lafayette, CO) (approximately 50 µg = 40 µL) was added dropwise and the mixture was carefully vortexed. Subsequently, this siRNA/DDAB mixture was incubated at room temperature for 1 hour in dark. The neutral lipid solution (i.e., containing POPC) was added dropwise and the mixture was vortexed carefully.

The lipid/siRNA was dialyzed in the dark against buffer (20 mM HEPES and 140 mM NaCl, pH 7.5) using the MiniLipoprep apparatus (product number 746300) with dialysis membranes of 5,000 Da MWCO (all purchased from Harvard Apparatus, Hollister, MA) per manufacturer's instructions, for approximately 60 hours changing the dialysis buffer at least once a day.

The lipid/siRNA was evaporated to a final volume of 100 µL (100 mM); 10 cycles repeated for freezing the lipid/siRNA in ethanol/dry ice mix for 4 min followed by thawing at 40°C for 2 min. A concentration of 20 mM was achieved by adding 400 µL of buffer (20 mM HEPES and 140 mM NaCl, pH 7.5). The lipid/siRNA complexes were then sequentially extruded using a mini-extruder (Product No. 610000, Avanti Polar Lipids, Alabaster, AL) with polycarbonate membranes serially of sizes 400 nm, 200 nm, and 100 nm (21 passages through the filter for each-sized filter), per the manufacturer's instructions. In brief, extrusions were performed with the mini-extruder sitting upon a heat-block heated to 40°C. When using the 400 nm and 200 nm membranes, two membranes sandwiched between 4 filter supports (2 on each side) were used, while only one membrane sandwiched between 4 filter supports (2 on each side) was used for extruding through the 100 nm membrane. The resultant liposomes produced were in the size range of 100 nm to 200 nm, as measured by the Partica LA-950 laser diffraction particle size analyzer (Jobin Yvon, Edison, NJ).

Sialic acid residues on the surface of liposomes were conjugated to a monoclonal antibody (ACL8971, Accurate Chemical & Scientific, Westbury, NY) targeting the transferrin receptor (TfR) that is highly expressed in the blood brain barrier (as well as elsewhere in the body, i.e., liver, lungs). This was done using a procedure using periodate oxidation adapted from Liposomes: A Practical Approach (2nd edition, 2003), edited by Vladimir Torchilin and Volkmar Weissig, Oxford University Press, Oxford, UK, Protocol 7, pages 202-203, and Heath et al., Biochem. Biophys. Acta, 640, 66 (1981).

Sodium periodate solution (0.1 mL, 0.2 M in water) was incubated with the liposome suspension (approximately 0.5 mL) in a brown vial at ambient temperature for 30 minutes. Sodium periodate and unencapsulated siRNA were removed by dialyzing with Spectra/Por Float-α-Lyzer (Spectrum Labs, Catalog No. 235-045, 25 kDa MWCO) overnight (approximately 24 hours) against borate buffer (1L, 20 mM sodium borate Na₂B₄O₇*10H₂O with 120 mM NaCl, pH 8.4). After dialysis, the material (approximately 0.6 mL) was transferred to a vial and mixed with 0.5 mL transferrin receptor antibody (ACL8971). Into this mixture, sodium cyanoborohydride solution (0.2 mL, 2 M in water) was added; the materials were shaken well and rocked overnight at 4°C.

The unconjugated TfR antibodies were removed by dialyzing against borate buffer (1L, 20 mM sodium borate Na₂B₄O₇*10H₂O with 120 mM NaCl, pH 8.4) at 4°C with Harvard Apparatus' FastDIALYZER with 0.05 µm polycarbonate membrane. The buffer was changed twice per day with dialysis performed for over 2 days so as to remove the residual unconjugated antibodies. Figure 1 depicts the siRNA/liposome complexes as punctate red fluorescent particles (signal emitting from the DY-547 red fluorescent label on siRNA shown as lighter spots in the image) when imaged under a fluorescent microscope.

The complete disclosure of all patents, patent applications, and publications, and electronically available material (e.g., GenBank amino acid and nucleotide sequence submissions; and protein data bank (pdb) submissions) cited herein are incorporated by reference. The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

## Claims

1. A liposome complex comprising:
a liposome having an exterior surface and an internal compartment;
a pharmaceutical agent comprising SiRNA associated with the liposome;
a sialic acid-containing molecule associated with the liposome; and
optionally a targeting agent.

2. The liposome complex of claim 1 which comprises a targeting agent, wherein the sialic acid-containing molecule forms a linker between the targeting agent and the liposome.

3. The liposome complex of claim 1, wherein the sialic acid-containing molecule comprises polysialic acid having a degree of polymerization of at least 8.

4. The liposome complex of any one of claims 1 to 3, wherein the liposome is prepared from phospholipids selected from the group consisting of phosphatidylserine, phosphatidylinositol, phosphatidylethanolamine, phosphatidylcholines, phosphatidylglycerol, phosphatidic acid, phosphatidylmethanol, cardiolipin, ceramide, cholesterol, cerebroside, lysophosphatidylcholine, D-erythrosphingosine, sphingomyelin, dodecyl phosphocholine, N-biotinyl phosphatidylethanolamine, and combinations thereof.

5. The liposome complex of any one of claims 1 to 4, wherein the pharmaceutical agent is located within the internal compartment of the liposome.

6. The liposome complex of any one of claims 1 to 5, wherein the sialic acid-containing molecule is attached at the external surface of the liposome.

7. The liposome complex of any one of claims 1 to 6, wherein at least one sialic acid-containing molecule comprises a lipid head-group and a sialic acid group.

8. The liposome complex of claim 7, wherein the lipid head-group is a phospholipid.

9. The liposome complex of any one of claims 1 to 8, wherein the sialic acid-containing molecule is selected from the group consisting of capsular polysialic acid, sialic acid-containing gangliosides, colominic acid, and combinations thereof.

10. The liposome complex of claim 9, wherein the sialic acid-containing molecule is a capsular polysialic acid.

11. The liposome complex of any one of claims 1 to 10, comprising two or more different pharmaceutical agents.

12. The liposome complex of any one of claims 1 to 11 which comprises a targeting agent, wherein the sialic acid-containing molecule forms a linker between the targeting agent and the external surface of the liposome.

13. The liposome complex of any one of the preceding claims, wherein the targeting agent is selected from the group consisting of peptides, peptide analogs, antibodies, antibody fragments, small organic or inorganic or organometallic molecules, and combinations thereof.

14. The liposome complex of claim 13, wherein the targeting agent is a compound or portion thereof that specifically recognizes receptors expressed in the brain microvasculature.

15. A pharmaceutical composition comprising the liposome complex of any one of claims 1 to 14 and an optional pharmaceutically acceptable carrier.

16. The pharmaceutical composition of claim 15, further comprising a pharmaceutical agent that is not a part of the liposome complex.

17. The pharmaceutical composition of claim 15 or claim 16, which crosslinks, gels, or changes in viscosity on or after administration to a subject.

18. The pharmaceutical composition of any one of claims 15 to 17, which provides for delayed or extended release of the liposome complex after administration to a subject.

19. The pharmaceutical composition of any one of claims 15 to 18, comprising two or more different liposome complexes, each containing different pharmaceutical agents and/or targeting agents.

20. A liposome complex or pharmaceutical composition as claimed in any one of claims 1 to 19 for use in a method of treating and/or preventing an affliction in a subject.

21. The liposome complex or pharmaceutical composition as claimed in claim 20, wherein the affliction is a psychiatric disorder, a neurological disorder, a disease affecting and/or originating in the brain, a disorders of the cardiac system, a disorder of the hepatic system, a disorder of the vascular system, a disorder of the orthopedic system, or combinations thereof.

22. A liposome complex or pharmaceutical composition as claimed in any one of claims 1 to 19 for use in a method of delivering a pharmaceutical agent to a subject.

23. The liposome complex or pharmaceutical composition as claimed in claim 22, wherein the liposome complex is administered to the subject for preferential uptake by an organ of interest.

24. A method of making a liposome complex as claimed in claim 1 or claim 2, the method comprising:
preparing a liposome having an exterior surface and an internal compartment, a pharmaceutical agent comprising an SiRNA associated therewith, and a sialic acid-containing molecule associated therewith; and
attaching a targeting agent to the sialic acid-containing molecule;
wherein the sialic acid-containing molecule forms a linker between the targeting agent and the liposome.

25. A method of making a liposome complex as claimed in claim 1 or claim 3, the method comprising:
preparing a liposome having an exterior surface and an internal compartment, a pharmaceutical agent comprising an SiRNA associated therewith, and a sialic acid-containing molecule associated therewith; and
optionally attaching a targeting agent to the sialic acid-containing molecule;
wherein the sialic acid-containing molecule comprises polysialic acid having a degree of polymerization of at least 8.

26. The method of claim 24 or claim 25, wherein preparing a liposome having an exterior surface and an internal compartment and a pharmaceutical agent associated therewith comprises encapsulating the pharmaceutical agent within the internal compartment of the liposome.

27. The method of claim 26, wherein preparing a liposome having an exterior surface and an internal compartment and a sialic acid-containing molecule associated therewith comprises attaching a sialic acid-containing molecule to the external surface of the liposome during or after encapsulation of the pharmaceutical agent.

28. The method of claim 27, further comprising attaching a targeting agent to the sialic acid-containing molecule before, during, or after attaching the sialic acid-containing molecule to the external surface of the liposome.

29. The method of claim 24, wherein preparing a liposome comprises forming the liposome from a sialic acid-containing molecule that includes a lipid group and a sialic acid group.

30. The method of claim 24, wherein preparing a liposome comprises forming the liposome from a sialic acid-containing molecule that does not include a lipid group.
